# EUROPEAN PATENT SPECIFICATION

(11) **EP 0 574 952 B1**
(45) Date of publication and mention of the grant of the patent: **10.04.2002**
(21) Application number: 93109812.3
(22) Date of filing: 18.06.1993
(51) Int. Cl.: C07D 207/27, C07C 233/07, C07C 231/02

(54) **Process for preparing 2-oxopyrrolidinylacetamide derivatives**
Verfahren zur Herstellung von 2-Oxopyrrolidinylacetamid-Derivaten
Procédé pour la préparation de dérivés 2-oxopyrrolidinylacétamide

(30) Priority: 19.06.1992 JP 16049692
(43) Date of publication of application: 22.12.1993
(60) Divisional application: 97120490.4
(73) Proprietor: DAIICHI PHARMACEUTICAL CO., LTD., Chuo-ku, Tokyo 103 (JP)
(72) Inventor: Kamihara, Shinji, c/o Daiichi Pharmac. Co., Ltd., Edogawa-ku, Tokyo (JP); Kaneuchi, Tohru, c/o Daiichi Pharmac. Co., Ltd., Edogawa-ku, Tokyo (JP); Uchiyama, Keiji, c/o Daiichi Pharmac. Co., Ltd., Edogawa-ku, Tokyo (JP); Terada, Tatsuya, c/o Daiichi Pharmac. Co., Ltd., Edogawa-ku, Tokyo (JP)
(74) Representative: Kinzebach, Werner, Dr.

(56) References cited:
- EP-A- 0 008 057
- EP-A- 0 065 804
- CH-A- 518 266
- FR-A- 2 216 267
- GB-A- 1 039 113
- GB-A- 2 053 909
- US-A- 2 863 752
- US-A- 3 647 876
- US-A- 3 867 446

## Description

The present invention relates to a process for preparing a pyrrolidinylacetamide derivative useful as an agent for improving cerebral functions.

Known processes for preparing pyrrolidinylacetamide derivatives include a process comprising reacting pyrrolidinylacetic acid or a reactive derivative thereof with an amine in an organic solvent in the presence of dicyclohexylcarbodiimide as disclosed in U.S. Patent 4,341,790 and JP-A-56-2960 (the term "JP-A" as used herein means an "unexamined published Japanese patent application").

The above process is not suited for production on an industrial scale due to disadvantages, such as use of expensive dicyclohexylcarbodiimide as a condensing agent, involvement of complicated steps for separation and removal of dicyclohexylurea which is a by-product from dicyclohexylcarbodiimide, poor overall yield. Moreover, the starting material, e.g., 2-oxo-1-pyrrolidinylacetic acid, should be produced by reacting 2-pyrrolidinone with methyl bromoacetate and hydrolyzing the resulting methyl 2-oxo-1-pyrrolidinylacetate, and purified by distilling the reaction mixture under high pressure and at high temperature. These complicated operations and low yields are disadvantageous for industrial production.

The preparation of N-substituted 2-chloroacetamides is described in various documents. For instance US 3,867,446 relates to the synthesis of N-alkyl-substituted chloroacetanilides, e.g. N-isopropyl-chloroacetanilide (see also FR-A-2,216,267). N,N-di-n-propyl-chloroacetamide is prepared in US 3,647,876.

US 2,863,752 and EP 0 008 057 A1 describe N-substituted α-halogenoacetanilides. While in US 2,863,752 chloroacetyl chloride is reacted with the appropriate N-substituted aniline EP 0 008 057 employs a process wherein the appropriate α-cloroacetanilide is reacted with the N-substituent. For example, 2-chloro-N-(2,6-dimethylphenyl)acetamide is converted to N-substituted α-halogenoacetanilides of the formula

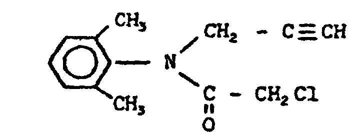

In CH 518 266 the same intermediate compound, i.e. 2-chloro-N-(2,6-dimethylphenyl)acetamide, is reacted with 1-methyl-2-phenylethylamine to give a compound of the formula

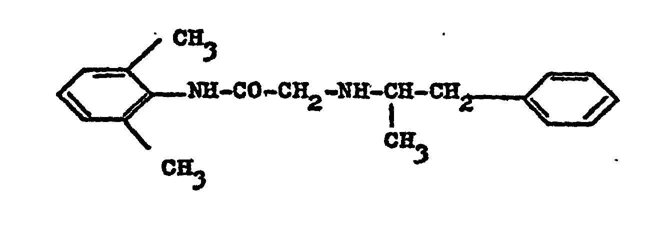

On the other hand, a process for preparing N-substituted lactams is known as desclosed in British Patent No. 1,039,113. This known process comprises reacting a N-unsubstituted lactam with an alkali metal hydride and then reacting the resulting alkali metal derivative with an appropriate ω-chloroalkylamide. The chloroacetanilide derivative used in the process is prepared by a manner as disclosed, for example, in Yakugaku Zasshi (Pharmacological Journal), vol. 99(2), pp. 146 to 154 (1979), which comprises reacting xylidine with monochloroacetyl chloride in a glacial acetic acid to obtain 2,6-dimethylmonochloroacetanilide in a 78 to 80% yield. However, these processes are still not satisfactory from the viewpoint of yield on an industrial scale.

An object of the present invention is to provide a process for preparing a pyrrolidinylacetamide derivative which is free from the above-mentioned disadvantages associated with the known process and excellent from the economical standpoint.

The base of the present invention consists in a reaction of a halogenoacetamide derivative and 2-pyrrolidinone, with various improvements added thereto, for example, an improvement in which the starting halogenoacetamide derivative is obtained in a high yield by reacting an amine and a halogenoacetyl chloride in a two-phase reaction system.

The present invention provides a process for preparing a 2-(1-pyrrolidinyl)acetamide derivative represented by formula (I):

R²-CH₂CONH-R¹ (I)

wherein R¹ represents a substituted or unsubstituted pyridyl group or a substituted phenyl group, which substituents are selected from the group consisting of a halogen atom, a trifluormethyl group, a nitro group, an acetyl group, an alkyl group, an alkoxy group, an alkylmercapto group, an amino group, a sulfonyl group, and an aminoethoxycarbonyl group; and R² represents a 2-oxo-1-pyrrolidinyl group, which may be substituted with a hydroxyl group, comprising reacting 2-pyrrolidinone, which may be substituted with a hydroxyl group, with an alcoholate to obtain a metal salt of said 2-pyrrolidinone and reacting the salt with a halogenoacetamide derivative represented by formula (II):

X-CH₂CONH-R¹ (II) (II)

wherein R¹ is as defined above; and X represents a halogen atom.

A specific embodiment of the invention is a process which comprises reacting an amine represented by formula (III):

NH₂-R¹ (III)

wherein R¹ is as defined above,
with a halogenoacetyl chloride in a two-phase reaction system composed of an aqueous layer and an organic solvent layer to obtain a halogenoacetamide derivative represented by formula (II):

X-CH₂CONH-R¹ (II)

wherein R¹ is as defined in Claim 1; and X represents a halogen atom,
and reacting the resulting halogenoacetamide derivative of formula (II) with 2-pyrrolidinone, which may be substituted with a hydroxyl group.

The 2-(1-pyrrolidinyl)acetamide derivative of formula (I) is a known compound disclosed in U.S. Patent 4,341,790 and JP-A-56-2960. Definitions of symbols used in formula (I) accord with those given in U.S. Patent 4,341,790 and JP-A-56-2960. That is, in addition to the definitions described above, the substituent which may be on the pyridyl or phenyl group as R¹ includes a halogen atom, a trifluoromethyl group, a nitro group, an acetyl group, an alkyl group, an alkoxy group, an alkylmercapto group, an amino group, a sulfonyl group, and an aminoethoxycarbonyl group, and the substituent which may be on the 2-oxo-1-pyrrolidinyl group as R² includes a hydroxyl group.

The "alkyl moiety" of the above substituents means a lower alkyl group having from 1 to 5 carbon atoms.

Of the compounds of formula (I), the most useful is N-(2,6-dimethylphenyl)-2-(2-oxo-1-pyrrolidinyl)-acetamide.

Organic solvents which can be used for formation of an aqueous/organic solvent two phase system where an amine and a halogenoacetyl chloride are reacted are selected from those capable of forming a separate phase when mixed with water or water having dissolved therein an inorganic base or a starting material and preferably those inert to the starting materials. Suitable organic solvents include chloroform, methylene chloride, dichloroethylene, toluene, xylene, ethyl acetate, t-butyl methyl ether, acetone, and acetonitrile. Toluene and xylene are particularly suitable from the viewpoint of recovery and reduced air pollution as well as reaction yield. It is preferable to use the solvent in an amount enough to dissolve the starting materials. However, it is not necessary to use it in an amount enough to dissolve completely the resulting halogenoacetylamide.

The halogenoacetyl chloride which can be used in the present invention is preferably chloroacetyl chloride, which gives a chloroacetamide derivative of formula (II).

It is preferable to make the aqueous layer weakly basic to basic for satisfactory progress of the reaction. To this effect, a base, such as sodium carbonate, sodium hydrogencarbonate, sodium hydroxide, potassium hydroxide, etc., is preferably added in at least an equivalent amount.

The molar ratio of the halogenoacetyl chloride to the amine is at least 1, and preferably 1.1 to about 1.5. The reaction product (II) can be isolated in a usual manner, for example, (1) by cooling the reaction mixture and collecting the product by filtration, or (2) by separating and concentrating the organic layer and collecting the product by filtration.

Though the intermediate of the formula (II) can be isolated as described above, it is advantageous to use the organic layer in the subsequent step without isolating such intermediate, since there are little by-products. Practically, the organic layer separated is azeotropically distilled under reduced pressure to about 1/2 to 1/3 by volume, thus water content in the organic solvent becomes within an allowable limit (about 0.05% v/v).

Before reacting the resulting halogenoacetamide derivative with substituted or unsubstituted 2-pyrrolidinone, it is necessary to treat the 2-pyrrolidinone with a base. Bases which can be used include strong bases, such as potassium hydride, sodium hydride, sodium amide, n-butyl lithium, t-butyl lithium, diethylamino lithium and alcoholates. Of these bases, it is preferred to use various alcoholates which are less expensive and less ignitable and to carry out the treatment under heating at about 100 to 150°C for 0.5 to 5 hours while driving the by-produced alcohol out of the system. In particular, sodium methylate and sodium ethylate are preferred because of cheapness and easy removal of by-produced methanol or ethanol. The amount of the-base to be used is preferably not more than an equimolar amount with the pyrrolidinone. Use of the base in excess tends to cause side reactions.

The treatment with a base is usually effected in an appropriate solvent which is unreactive with the base. Examples of suitable solvents are toluene, xylene, chloroform, methylene chloride, dichloroethylene, and t-butyl methyl ether. The treatment is preferably conducted in a water-free system because water, if present, may react with the base or may interfere with the treatment.

The halogenoacetamide derivative is added to the pyrrolidinone compound having been treated with a base, and the mixture is allowed to react at a temperature of from 0 to 100°C, and preferably from 40 to 80°C, to obtain the desired pyrrolidinylacetamide derivative. The halogenoacetamide is usually used in an amount not more than an equimolar amount with the base used in the above-mentioned treatment. In carrying out the reaction, it is advantageous from the operation standpoint to use the same solvent as used in the above-mentioned treatment with a base.

The reaction product can easily be isolated by cooling the reaction mixture by addition of water to crystallize the product. This isolation step is also an inventive aspect of the present invention.

The present invention will now be illustrated in greater detail with reference to Examples, but it should be understood that the present invention is not construed as being limited thereto.

### EXAMPLE 1

### Preparation of 2-Chloro-N-(2,6-dimethylphenyl)acetamide

In 3 ℓ of toluene was dissolved 182 g of 2,6-xylidine, and in 1.5 ℓ of water was dissolved 159 g of sodium carbonate. The two solutions were combined, and 203 g of chloroacetyl chloride was added thereto dropwise over 1.5 hours at an inner temperature of from 20 to 35°C, followed by stirring at that temperature for 1.5 hours. The reaction mixture was cooled with ice, and the precipitated crystals were collected by filtration and dried under reduced pressure to obtain 282 g (95%) of the titled compound.

| | |
|---|---|
| Melting point | 148-148.5°C |
| ¹H-NMR (CDCℓ₃) | 2.24 (6H, s), 4.24 (2H, s), 7.16 (3H, s), 7.9 (1H, br s) |

### EXAMPLE 2

### Preparation of 2-Chloro-N-(2,6-dimethylphenyl)acetamide

In 4 ℓ of 1,2-dichloroethylene was dissolved 182 g of 2,6-xylidine, and 1.6 ℓ of a 1N sodium hydroxide aqueous solution was added thereto, followed by stirring. To the mixture was added dropwise 203 g of chloroacetyl chloride over 1.5 hours at an inner temperature between 20 and 35°C. The mixture was stirred at that temperature for 1.5 hours. The reaction mixture was separated into two layers, and the organic layer was concentrated under reduced pressure. The precipitate was collected by filtration and dried under reduced pressure to obtain 282 g (95%) of the titled compound.
Melting point: 148-148.5°C
¹H-NMR data agreed with those of Example 1.

### EXAMPLE 3

### Preparation of N-(2,6-Dimethylphenyl)-2-(2-oxo-1-pyrrolidinyl)acetamide

In 300 mℓ of toluene was suspended 9.2 g of 60% sodium hydride in a nitrogen stream, and 21.3 g of 2-pyrrolidinone was slowly added dropwise to the suspension at an inner temperature controlled at 40°C or lower. After stirring the mixture for 2 hours, 19.7 g of 2-chloro-N-(2,6-dimethylphenyl)acetamide was added thereto, and the mixture was allowed to react at 60 to 70°C for 2 hours. To the reaction mixture was added 50 mℓ of water, followed by allowing to cool with stirring. The precipitated crystals were collected by filtration and dried under reduced pressure to obtain 22.2 g (90%) of the titled compound.

| | |
|---|---|
| Melting point | 153-153.5°C |
| ¹H-NMR (CDCℓ₃) | 1.9-2.6 (4H, m), 2.18 (6H, s), 3.57 (2H, t, J=6.8Hz), 4.07 (2H, s), 7.06 (3H, s), 7.9 (1H, br s) |

### EXAMPLE 4

### Preparation of N-(2,6-Dimethylphenyl)-2-(2-oxo-1-pyrrolidinyl)acetamide

In 30 mℓ of t-butyl methyl ether was suspended 897 mg of sodium amide in a nitrogen stream, and 2.13 g of 2-pyrrolidinone was slowly added thereto dropwise, followed by refluxing for 2 hours. After allowing to cool, 1.97 g of 2-chloro-N-(2,6-dimethylphenyl)acetamide was added thereto, followed by refluxing for 2 hours. To the reaction mixture was added 50 mℓ of water, and the mixture was allowed to cool with stirring. The thus formed crystals were collected by filtration and dried under reduced pressure to obtain 2.19 g (89%) of the titled compound.
Melting point: 151-152.5°C

### EXAMPLE 5

### Preparation of N-(2,6-Dimethylphenyl)-2-(2-oxo-1-pyrrolidinyl)acetamide

In 300 mℓ of toluene was suspended 9.0 g of sodium amide in a nitrogen stream, and 21.3 g of 2-pyrrolidinone was slowly added thereto dropwise. After stirring the mixture at 60 to 70°C for 2 hours, 19.7 g of 2-chloro-N-(2,6-dimethylphenyl)acetamide was added thereto, followed by allowing to react at 60 to 70°C for 2 hours. To the reaction mixture was added 50 mℓ of water, and the mixture was allowed to cool while stirring. The precipitated crystals were recovered by filtration and dried under reduced pressure to obtain 22.4 g (91%) of the titled compound.
Melting point: 152-152.5°C

### EXAMPLE 6

### Preparation of N-(2,6-Dimethylphenyl)-2-(2-oxo-1-pyrrolidinyl)acetamide

In 5 ℓ of toluene was suspended 203 g of sodium methylate, and 511 g of 2-pyrrolidinone was added thereto. The mixture was slowly heated up to 100 to 110°C, at which it was allowed to react for 3 hours. During the reaction, about 300 mℓ of toluene was distilled off. After allowing to cool, 296 g of 2-chloro-N-(2,6-dimethylphenyl)acetamide was added thereto to react at 60 to 70°C for 2 hours. To the reaction mixture was added 300 mℓ of water, followed by allowing to cool with stirring. The crystals formed were collected by filtration and dried under reduced pressure to obtain 332 g (90%) of the titled compound.
Melting point: 152.5-153°C

### EXAMPLE 7

### Preparation of N-(2,6-Dimethylphenyl)-2-(2-oxo-1-pyrrolidinyl)acetamide

In 5 ℓ of toluene was dissolved 182 g of 2,6-xylidine, and in 1.5 ℓ of water was dissolved 159 g of sodium carbonate. The two solutions were combined, and 203 g of chloroacetyl chloride was added thereto dropwise over 1.5 hours at an inner temperature of from 20 to 35°C, followed by stirring at that temperature for 1.5 hours. The reaction mixture was heated to about 70°C to dissolve the precipitated crystals and then separated into two layers. The organic layer was concentrated under reduced pressure to about 500 mℓ. The resulting suspension of 2-chloro-N-(2,6-dimethylphenyl)acetamide was allowed to cool.

Separately, 203 g of sodium methylate was suspended in 4 ℓ of toluene, and 511 g of 2-pyrrolidinone was added thereto. The mixture was slowly heated up to 100 to 110°C, at which it was allowed to react for 3 hours. During the reaction, about 300 mℓ of toluene was distilled off. After allowing to cool, the above prepared suspension of 2-chloro-N-(2,6-dimethylphenyl)acetamide was added thereto to react at 60 to 70°C for 2 hours. To the reaction mixture was added 300 mℓ of water, followed by allowing to cool with stirring. The crystals formed were collected by filtration and dried under reduced pressure to obtain 336 g (91%) of the titled compound. Melting point: 152-153°C

While the invention has been described in detail and with reference to specific examples thereof, it will be apparent to one skilled in the art that various changes and modifications can be made therein without departing from the spirit and scope thereof.

## Claims

1. A process for preparing a 2-(1-pyrrolidinyl)-acetamide derivative represented by formula (I):
R²-CH₂CONH-R¹ (I)
wherein R¹ represents a substituted or unsubstituted pyridyl group or a substituted phenyl group, which substituents are selected from the group consisting of a halogen atom, a trifluormethyl group, a nitro group, an acetyl group, an alkyl group, an alkoxy group, an alkylmercapto group, an amino group, a sulfonyl group, and an aminoethoxycarbonyl group; and R² represents a 2-oxo-1-pyrrolidinyl group, which may be substituted with a hydroxyl group, comprising reacting 2-pyrrolidinone, which may be substituted with a hydroxyl group, with an alcoholate to obtain a metal salt of said 2-pyrrolidinone and reacting the salt with a halogenoacetamide derivative represented by formula (II):
X-CH₂CONH-R¹ (II)
wherein R¹ is as defined above; and X represents a halogen atom.

2. A process as claimed in Claim 1, wherein R² is a 2-oxo-1-pyrrolidinyl group, X is a chlorine atom, R¹ is a 2,6-dimethylphenyl group, and said alcoholate is sodium methylate or sodium ethylate.

3. A process as claimed in Claim 1 or Claim 2, comprising reacting an amine represented by formula (III):
NH₂-R¹ (III)
wherein R¹ is as defined in Claim 1,
with a halogenoacetyl chloride in a two-phase reaction system composed of an aqueous layer and an organic solvent layer to obtain a halogenoacetamide derivative represented by formula (II):
X-CH₂CONH-R¹ (II)
wherein R¹ is as defined in Claim 1; and X represents a halogen atom,
and reacting the resulting halogenoacetaaide derivative of formula (II) with 2-pyrrolidinone, which may be substituted with a hydroxyl group.

4. A process as claimed in Claim 3, wherein said halogenoacetyl chloride is chloroacetyl chloride, and said halogenoacetamide derivative is a chloroacetamide derivative.

5. A process as claimed in Caim 3, wherein said 2-pyrrolidinone is unsubstituted, said halogenoacetyl chloride is chloroacetyl chloride, and said amine is 2,6-xylidine.

6. A process as claimed in Claim 4, wherein said 2-pyrrolidinone is unsubstituted, and said amine is 2,6-xylidine.

7. A process as claimed in any of Claims 3 to 6, wherein the halogenoacetamide derivative is used without isolating it.

## Patentansprüche

1. Verfahren zur Herstellung eines 2-(1-pyrrolidinyl)acetamid-Derivats der Formel (I):
R²-CH₂CONH-R¹ (I)
worin R¹ für eine substituierte oder nichtsubstituierte Pyridylgruppe oder eine substituierte Phenylgruppe steht, wobei die Substituenten ausgewählt sind unter einem Halogenatom, einer Trifluormethylgruppe, einer Nitrogruppe, einer Acetylgruppe, einer Alkylgruppe, einer Alkoxygruppe, einer Alkylmercaptogruppe, einer Aminogruppe, einer Sulfonylgruppe und einer Aminoethoxycarbonylgruppe; und R² für eine 2-Oxo-1-pyrrolidinylgruppe steht, die mit einer Hydroxylgruppe substituiert sein kann,
wobei man 2-Pyrrolidinon, das mit einer Hydroxylgruppe substituiert sein kann, mit einem Alkoholat umsetzt, so dass ein Metallsalz des 2-Pyrrolidinons erhalten wird, und das Salz mit einem Halogenacetamid-Derivat der Formel (II):
X-CH₂CONH-R¹ (II)
worin R¹ wie oben definiert ist; und X ein Halogenatom ist, umsetzt.

2. Verfahren nach Anspruch 1, wobei R² eine 2-Oxo-1-pyrrolidinylgruppe ist, X ein Chloratom ist, R¹ eine 2,6-Dimethylphenylgruppe ist, und das Alkoholat Natriummethylat oder Natriumethylat ist.

3. Verfahren nach Anspruch 1 oder Anspruch 2, wobei man ein Amin der Formel (III):
NH₂-R¹ (III)
worin R¹ wie in Anspruch 1 definiert ist,
mit einem Halogenacetylchlorid in einem 2-phasigen Reaktionssystem, das aus einer wässrigen Schicht und einer organischen Lösungsmittelschicht zusammengesetzt ist, umsetzt, so dass ein Halogenacetamid-Derivat der Formel (II):
X-CH₂CONH-R¹ (II)
worin R¹ wie in Anspruch 1 definiert ist; und X ein Halogenatom ist, erhalten wird, und das resultierende Halogenacetamid-Derivat der Formel (II) mit 2-Pyrrolidinon, das mit einer Hydroxylgruppe substituiert sein kann, umsetzt.

4. Verfahren nach Anspruch 3, wobei das Halogenacetylchlorid Chloracetylchlorid ist und das Halogenacetamid-Derivat ein Chloracetamid-Derivat ist.

5. Verfahren nach Anspruch 3, wobei das 2-Pyrrolidinon nicht substituiert ist, das Halogenacetylchlorid Chloracetylchlorid ist und das Amin 2,6-Xylidin ist.

6. Verfahren nach Anspruch 4, wobei das 2-Pyrrolidinon nicht substituiert ist und das Amin 2,6-Xylidin ist.

7. Verfahren nach einem der Ansprüche 3 bis 6, wobei das Halogenacetamid-Derivat verwendet wird, ohne es zu isolieren.

## Revendications

1. Procédé pour la préparation d'un dérivé 2-(1-pyrrolidinyl)-acétamide représenté par la formule (I) :
R²-CH₂CONH-R¹ (I)
où R¹ représente un groupe pyridyle substitué ou non substitué ou un groupe phényle substitué, lesquels substituants sont choisis parmi un atome d'halogène, un groupe trifluorométhyle, un groupe nitro, un groupe acétyle, un groupe alkyle, un groupe alcoxy, un groupe alkylmercapto, un groupe amino, un groupe sulfonyle et un groupe aminoéthoxycarbonyle ; et R² représente un groupe 2-oxo-1-pyrrolidinyle, lequel peut être substitué avec un groupe hydroxyle, comprenant la réaction de 2-pyrrolidinone, laquelle peut être substituée avec un groupe hydroxyle, avec un alcoolate pour obtenir un sel de métal de ladite 2-pyrrolidinone et la réaction du sel avec un dérivé halogénoacétamide représenté par la formule (II) :
X-CH₂CONH-R¹ (II)
où R¹ est comme défini ci-dessus ; et X représente un atome d'halogène.

2. Procédé selon la revendication 1, dans lequel R² est un groupe 2-oxo-1-pyrrolidinyle, X est un atome de chlore, R¹ est un groupe 2,6-diméthylphényle et ledit alcoolate est le méthylate de sodium ou l'éthylate de sodium.

3. Procédé selon la revendication 1 ou la revendication 2 comprenant la réaction d'une amine représentée par la formule (III) :
NH₂-R¹ (III)
où R¹ est comme défini dans la revendication 1,
avec un chlorure d'halogénoacétyle dans un système réactionnel à deux phases constitué d'une couche aqueuse et d'une couche de solvant organique pour obtenir un dérivé halogénoacétamide représenté par la formule (II) :
X-CH₂CONH-R¹ (II)
où R¹ est comme défini dans la revendication 1 ; et X représente un atome d'halogène,
et la réaction du dérivé halogénoacétamide résultant de la formule (II) avec de la 2-pyrrolidinone, laquelle peut être substituée avec un groupe hydroxyle.

4. Procédé selon la revendication 3, dans lequel ledit chlorure d'halogénoacétyle est le chlorure de chloroacétyle et ledit dérivé halogénoacétamide est un dérivé de chloroacétamide.

5. Procédé selon la revendication 3, dans lequel ladite 2-pyrrolidinone n'est pas substituée, ledit chlorure d'halogénoacétyle est le chlorure de chloroacétyle et ladite amine est la 2,6-xylidine.

6. Procédé selon la revendication 4, dans lequel ladite 2-pyrrolidinone n'est pas substituée et ladite amine est la 2,6-xylidine.

7. Procédé selon l'une quelconque des revendications 3 à 6, dans lequel le dérivé halogénoacétamide est utilisé sans l'isoler.
